# FASCICULE DE BREVET EUROPEEN

(11) **EP 0 495 861 B1**
(45) Date de publication et mention de la délivrance du brevet: **26.05.1993**
(21) Numéro de dépôt: 90915491.6
(22) Date de dépôt: 09.10.1990
(51) Int. Cl.: A61F 2/02

(54) **FILTRE ANTI-EMBOLIE PULMONAIRE ET KIT DE STOCKAGE ET DE POSE DE CE FILTRE**
ANTIEMBOLIEFILTER FÜR LUNGEN SOWIE KIT ZUM AUFBEWAHREN UND ANWENDEN DIESES FILTERS
ANTI-PULMONARY EMBOLISM FILTER AND KIT FOR STORING AND APPLYING SUCH FILTER

(30) Priorité: 09.10.1989 FR 8913160; 14.12.1989 FR 8916538
(43) Date de publication de la demande: 29.07.1992
(73) Titulaire: FONDATION DE L'AVENIR POUR LA RECHERCHE MEDICALE APPLIQUEE, F-75013 Paris (FR); ASSOCIATION POUR LA RECHERCHE EN IMAGERIE DE L'HOPITAL ANTOINE BECLERE, F-92140 Clamart (FR); DIBIE, Alain, F-75015 (FR); MUSSET, Dominique, F-92500 Rueil (FR)
(72) Inventeur: DIBIE, Alain, F-75015 Paris (FR); MUSSET, Dominique, F-92500 Rueil (FR)
(74) Mandataire: Martin, Jean-Jacques
(86) Numéro de dépôt international: FR9000721
(87) Numéro de publication internationale: WO9104716

(56) Documents cités:
- EP-A- 0 121 447
- EP-A- 0 323 333
- DE-A- 3 203 410
- FR-A- 2 616 666

## Description

La présente invention a pour objet un filtre anti-embolie pulmonaire et un kit de présentation et mise en place de ce filtre.

On connait déjà différents types, effectivement utilisés, de filtres destinés à être mis en place dans la veine cave inférieure afin d'y arrêter la migration vers le coeur droit de caillots provenant des veines des membres inférieurs ou du petit bassin.

On peut, en particulier, citer les filtres connus sous les noms de Mobin-Uddin, Greenfield, Gunther et L.G. Medical.

Tous ces types de filtres ont en commun d'avoir des structures de quasi-révolution, cylindriques ou coniques, qui doivent être repliées dans un fourreau poussé ensuite dans la lumière d'un cathéter vers la zone d'implantation. Leur fourreau d'introduction de diamètre égal ou supérieur à trois millimètres nécessitent des veines de gros calibres comme la veine jugulaire interne ou la veine fémorale.

Une fois en place dans la veine cave inférieure, ces filtres sont alors libérés de leur fourreau et leurs structures se déploient en élargissant modérément la section de la veine cave. Ils sont encombrants, difficiles à mettre en place, et quelquefois traumatisants pour les tissus de la veine cave dans lesquels ils viennent s'ancrer en plusieurs points d'accrochage. Les mauvaises positions et les migrations de ces filtres ne sont pas exceptionnelles.

Un autre type de filtre a été proposé dans le document FR-A-2541901. Selon ce document le filtre doit être réalisé à l'aide d'un fil élastique conformé pour présenter de manière permanente des ondulations successives dans des plans différents. On profiterait alors de l'élasticité de ce fil pour le mettre momentanément en forme rectiligne à l'intérieur d'un cathéter et le véhiculer ainsi vers la zone d'implantation. Arrivé en cette zone, et poussé hors du cathéter et libéré ainsi de celui-ci, il reprendrait sa forme initiale ondulée et viendrait s'appuyer en plusieurs points des plans différents précités contre les parois de la veine cave.

Cette proposition n'a toutefois pu faire l'objet de réalisations pratiques acceptables médicalement et a donc été abandonnée.

Un autre type de filtre a été proposé dans le document EP-A-323 333, sur lequel se basent les préambules des revendications indépendantes 1 et 20.

Selon ce document le filtre est réalisé en un fil métallique élastique à effet de ressort. Sa forme géométrique particulière détermine deux boucles en forme d'éllipses d'axes perpendiculaires et égaux coplanaires, donnant quatre points de contacts permanents avec la paroi de la veine cave dans laquelle il est implanté.

Ce dernier type de filtre a effectivement fait l'objet de réalisation et expérimentation sur l'animal, puis sur l'homme. Toutefois, de telles difficultés d'ordre médical sont apparues, tant sur la procédure de mise en place du filtre que sur son mode de fonctionnement une fois en place, que la généralisation d'emploi de ce filtre ne peut être recommandée médicalement.

Plus précisément, une première difficulté réside en ce que le filtre, qui a initialement la forme précitée en double ellipses d'axes perpendiculaires, après sa mise en forme momentanée rectiligne à l'intérieur du cathéter de mise en place, risque parfois, lors de sa libération hors du cathéter, à l'intérieur de la veine cave inférieure, de ne pas reprendre sa forme initiale. Cet inconvénient résulte des parties quasi rectilignes assez longues qui relient les parties quasi circulaires des quasi ellipses allongées. Ainsi, il y a des risques inacceptables de voir le filtre se retrouver en forme de S allongé ou de 8 et non plus en ellipses croisées. L'appui contre les parois de la veine cave en quatre points équilibrés n'est plus obtenu et, de ce fait, la veine n'est pas suffisamment aplatie, de sorte que le filtre peut migrer en se déplaçant le long de la veine après sa mise en place.

Par ailleurs, les plans de ces ellipses sont quasi coplanaires et l'effet de filtre n'est garanti que si les quatre points d'appui du filtre contre la paroi de la veine cave sont en bonne position, ce qui n'est déjà pas garanti comme on l'a exposé ci-dessus d'une part, et aplatissent très fortement la veine cave pour réduire fortement la section droite de la veine et obtenir ainsi l'effet de filtre, d'autre part.

Enfin, une extrémité du filtre est équipé d'une pointe acérée inclinée par rapport au plan des ellipses et servant de point d'ancrage dans la veine cave. Cette pointe acérée présente deux inconvénients : elle rend délicate son introduction dans le cathéter, d'une part, et risque de traumatiser ou même perforer la paroi de la veine, d'autre part.

La présente invention a pour but d'éliminer les inconvénients des filtres antérieurs, et accessoirement de proposer un nouveau kit de présentation et mise en place.

Dans toute la suite de ce texte, on utilisera, selon la convention médicale habituelle, l'adjectif "proximale" pour qualifier la partie de tout élément introduit dans le système circulatoire sanguin qui se trouve être la plus proche du point de ce système où doit être amené cet élément, et l'adjectif "distale" pour la partie de cet élément qui en est la plus éloignée.

Le filtre anti embolie pulmonaire conforme à la présente invention est réalisé en un fil élastique à effet de ressort rémanent conformé en spirale, et caractérisé en ce que la spirale comprend trois spires non jointives sensiblement circulaires dont la spire médiane a un diamètre supérieur à ceux des deux autres spires et que ce diamètre est choisi voisin de la valeur de la demi-circonférence de la veine cave dans la zone où le filtre doit être implanté pour assurer optimalement la fonction de maintien en place du filtre par aplatissement de la veine cave.

Cette conformation, caractéristique principale de l'invention, du filtre en spirale à trois spires non jointives a pour avantage essentiel que lors de sa libération hors du cathéter, à l'intérieur duquel il a été véhiculé sous forme transitoire rectiligne, le filtre reprend tout naturellement sa forme initiale puisque le filtre ne comporte que des parties curvilignes sensiblement de même courbure.

En second lieu, le rayon plus grand de la spire médiane étant choisi voisin de la valeur de la demi-circonférence de la veine cave c'est cette spire médiane qui, pour l'essentiel, assure la fonction de maintien en place du filtre dans la veine cave puisqu'elle vient s'appuyer fortement contre la paroi de cette veine cave le long de segments opposés pratiquement quart circulaires. On obtient ainsi, en toute certitude et sécurité, une mise en place correcte puis stable du filtre par aplatissemeat de la veine cave. Par ailleurs cet aplatissement de la veine cave est également favorable pour assurer la fonction de filtrage puisque la section de la veine cave dans laquelle le sang circule est ainsi réduite fortement. Les deux autres spires de la spirale à spires non jointives, donc non coplanaires avec la spire médiane, complètent ensuite optimalement cette fonction de filtrage dans la veine cave puisque le flux sanguin traversant ce filtre rencontre ainsi sur son parcours six éléments filaires semi-circulaires.

Selon une autre caractéristique l'extrémité proximale du filtre est munie d'un élément ovoïde radio-opaque et l'extrémité distale du filtre est munie d'un petit cylindre radio-opaque à bout libre arrondi.

Comme on le décrira ci-après plus en détail, ces extrémités et éléments radio-opaques permettent de contrôler par radiographie le transfert du filtre vers sa zone d'implantation puis sa mise en position correcte. L'élément ovoïde sert éventuellement au retrait ultérieur du filtre si on le désire.

Selon une autre caractéristique, l'ensemble du fil du filtre et de ses extrémités conformées comme précisé ci-dessus est revêtu d'une mince couche d'or afin d'éviter tout phénomène de corrosion, d'une part, et de garantir la bio-compatibilité du filtre mis en place à l'intérieur d'une veine cave, d'autre part.

Par ailleurs, la configuration en spirale du filtre, c'est-à-dire toute en courbures, permet de concevoir un kit de présentation et mise en place du filtre particulièrement adapté aux conditions de mise en oeuvre du filtre en milieu médical.

La présente invention a donc également pour objet un tel kit comprenant :
- un catheter,
- un poussoir constitué d'un embout rigide présentant, en son extrémité proximale arrondie, une encoche longitudinale pour recevoir l'extrémité distale du fil élastique du filtre, puis un logement pour recevoir le petit cylindre distal du filtre, l'embout rigide étant solidaire en son extrémité distale d'un câble et
- des moyens de bridage comprenant un conteneur apte à recevoir le poussoir muni de l'extrémité distale du filtre.

caractérisé par le fait que :
- le cathéter est terminé en son extrémité distale par un embout muni de moyens de raccordement rapide,
- le cable fixé à l'extrémité distale de l'embout rigide a une longueur au moins égale à la somme de la longueur du cathéter et de celle du filtre, et par le fait que le kit comprend en outre :
- un tube élastique transparent de même configuration en spirale que le filtre, d'une longueur au moins égale à celle du filtre et lui servant de conteneur de stockage et de présentation, ce tube étant équipé en son extrémité proximale de premiers moyens de raccordement rapide destinés à coopérer avec ceux précités du cathéter.

Selon une première variante de réalisation, les moyens de bridage sont constitués d'un boîtier rigide cylindrique formé de deux hémicylindres identiques reliés par une charnière, et destiné à recevoir l'assemblage de l'extrémité distale du filtre mise en place dans le logement de l'embout rigide du poussoir, l'extrémité proximale de ce boîtier étant muni de moyens de raccordement rapide coopérant avec des seconds moyens de raccordement rapide prévus à l'extrémité distale du tube.

Le boîtier rigide faisant partie de ce kit permet d'assembler l'extrémité distale du filtre et le poussoir associés juste au niveau de l'extrémité distale du tube de stockage du filtre. Ensuite, lorsque l'on actionne le poussoir, cet assemblage : poussoir et extrémité distale du filtre, sort du boîtier pour rentrer en premier dans le tube élastique transparent, puis dans le cathéter et il est ainsi maintenu en l'état assemblé jusqu'à sa sortie au niveau de l'extrémité proximale du cathéter.

Ainsi, la libération de la dernière spire du filtre a lieu dès cette sortie du cathéter, ce qui impose de contrôler avec une très grande précision la position de l'extrémité proximale du cathéter à l'intérieur de la veine cave inférieure, ce qui peut parfois être difficile.

Selon une seconde variante, les moyens de bridage sont constitués d'une gaine comportant une première partie tubulaire souple, de calibre interne et de longueur adaptés pour que le câble du poussoir puisse y être guidé et y glisser librement et une seconde partie rigide en forme de cloche tubulaire cylindrique susceptible, d'une part, de recevoir l'extrémité distale du filtre logée manuellement dans le poussoir à embout rigide et, d'autre part, d'être engagée manuellement dans l'extrémité distale du tube élastique transparent, le diamètre maximal extérieur de la gaine étant légèrement inférieur au diamètre interne du cathéter, et des moyens étant prévus pour pouvoir interdire sélectivement la sortie du poussoir de l'extrémité de la gaine.

Cette seconde variante permet de réaliser avec plus de facilité et de précision l'assemblage, puis le désassemblage du filtre et du poussoir.

En effet cette seconde variante permet de faire progresser l'ensemble de la gaine contenant le câble poussoir accouplé avec le filtre à l'intérieur du cathéter, puis dans la lumière de la veine cave, sans qu'ils se désunissent.

De la sorte, la mise en place du filtre dans cette veine cave s'opère en deux phases : au cours de la première, l'extrémité proximale du cathéter est mise en place dans la région choisie de la veine cave en étant suivie radiologiquement en raison de ses marques radio-opaques avec une première approximation suffisante ; puis, au cours d'une seconde phase, l'extrémité proximale radio-opaque de la gaine précitée est mise en une position très précise dans la veine cave. On autorise alors la progression du câble dans la gaine et une poussée supplémentaire du câble poussoir à l'intérieur de cette gaine permet la libération de l'extrémité distale du filtre qui parachève la mise en place du filtre.

Selon une autre caractéristique supplémentaire d'un kit selon l'une ou l'autre des variantes de réalisation proposées ci-dessus, l'extrémité proximale du cathéter est munie de deux anneaux espacés plus radio-opaques que le cathéter et permettant de mesurer le diamètre de la veine cave dans la zone où doit être mis en place le filtre pour pouvoir choisir en conséquence la valeur du diamètre de la spire médiane.

La présente invention a encore pour objet de compléter le kit en y incluant des moyens permettant à tout moment de récupérer le filtre après que celui-ci ait été entièrement libéré dans la lumière de la veine cave inférieure.

Il peut, en effet, être nécessaire de pouvoir procéder à cette récupération du filtre après sa mise en place, même correcte, pour des motifs médicaux ne mettant pas en cause la procédure de mise en place du filtre ni son efficacité.

Par exemple, il peut être judicieux de récupérer ce filtre un certain temps après sa mise en place, après qu'il ait joué son rôle anti-embolique et que les risques d'embolie pulmonaire aient disparu. Cela peut se produire dans le cas de jeunes accidentés de la circulation qui peuvent présenter initialement de tels risques.

A cet effet, l'invention propose d'adjoindre au kit précité un guide à crochet de récupération du filtre caractérisé en ce qu'il est constitué d'un guide souple pouvant coulisser librement dans un cathéter et terminé par un crochet rigide dans l'axe du guide et ayant une forme d'épingle à cheveux.

Pour récupérer un filtre, on fait parvenir l'extrémité proximale du cathéter, logeant le guide souple à crochet, dans la veine cave où est implanté le filtre à récupérer, à proximité de ce dernier. En poussant le guide, on fait sortir le crochet de l'extrémité du cathéter et on le pousse jusqu'à dépasser le filtre. Au cours d'un mouvement inverse, le crochet attrape une ou plusieurs spires. Une traction continue, du fait de l'élasticité du filtre, fait entrer celui-ci, par déformation, dans le cathéter, pour son retrait.

Selon une réalisation particulière, la branche libre du crochet rigide se termine par une extrémité souple inclinée vers l'extérieur' du crochet. Cela permet d'avoir une ouverture élargie du crochet pour aller attraper le nombre maximal de spires du filtre à retirer.

Selon une variante les moyens de retrait du filtre peuvent être formés d'une pince de retrait présentant en une extrémité deux mâchoires articulées, radio-opaques arrondies et creuses susceptibles d'être ouvertes ou fermées, par exemple par commande par un câble, pour pouvoir capter et tirer une extrémité de filtre.

En position d'ouverture, on peut en effet venir entourer l'une ou l'autre des extrémités d'un filtre en place que l'on souhaite retirer. On referme alors les mâchoires pour retirer par traction le filtre qui est véhiculé en retour dans un cathéter de retrait.

La description suivante de réalisations particulières des moyens de l'invention fera encore mieux comprendre les caractéristiques et avantages de celle-ci.

Cette description sera faite en référence aux dessins annexés, sur lesquels :
- la figure 1 représente, en perspective, le filtre en spirale à trois spires non jointives avec ses deux extrémités proximale et distale,
- la figure 2 est une vue de dessus de ce filtre supposé placé à plat sur une surface horizontale,
- la figure 3 est une vue schématique latérale de ce filtre permettant de bien illustrer les caractéristiques spécifiques géométriques de conformation des spires du filtre,
- la figure 4 illustre la forme transitoire rectiligne du filtre à l'intérieur d'un cathéter,
- les figures 5 et 6 illustrent le mode de transfert puis de largage d'un filtre à sa position dans une veine cave,
- les figures 7 et 8 représentent respectivement en coupe transversale de la veine cave et en vue latérale longitudinale de celle-ci, la forme que prend le filtre en spirale lorsqu'il est positionné dans une veine cave,
- la figure 9 représente un poussoir pour faire avancer le filtre dans un cathéter,
- la figure 10 représente un boîtier permettant d'assembler le filtre avec le poussoir,
- la figure 11 représente l'ensemble monté des quatre composants d'un kit de stockage, présentation et utilisation d'un filtre selon l'invention, conforme à la première variante précitée,
- la figure 12 représente la seconde variante de réalisation d'un tel kit,
- la figure 13 représente un guide à crochet de retrait de filtre selon l'invention,
- les figures 14 à 18 illustrent les modes d'utilisation et de fonctionnement de ce guide à crochet, et
- les figures 19 et 20 illustrent une pince à retrait selon l'invention et son mode de fonctionnement.

La description qui va suivre concernera successivement :
1) les caractéristiques de fil et de cathéter à utiliser pour conformer un filtre selon l'invention,
2) la conformation du filtre lui-même et de ses extrémités et les effets de la mise en place d'un filtre dans une veine cave,
3) la structure et l'utilisation des deux variantes de kit de stockage, présentation et utilisation de filtre,
4) des moyens de retrait du filtre.

### 1. CARACTERISTIQUES DE FIL ET DE CATHETER.

Le filtre anti-embolie pulmonaire est réalisé en un fil élastique, très préférentiellement métallique. Ce fil doit être non magnétisable pour permettre sans dommage au porteur d'un filtre d'être soumis à un examen RMN ou IRM (imagerie à résonance magnétique). Ce fil doit aussi être radio-opaque pour pouvoir être visible lors d'un examen aux rayons X.

Le fil peut être formé de tout matériau élastique approprié connu de l'homme de l'art.

A titre d'exemple non limitatif le fil peut être en un alliage choisi dans le groupe comprenant les alliages suivants: 1) cuivre et nickel et aluminium, 2) cuivre et zinc et aluminium, 3) cuivre et zinc et aluminium et nickel, 4) cuivre et étain et nickel.

Un revêtement final d'une mince couche de matériau bio-compatible, de préférence d'or, est effectué pour éviter des phénomènes de corrosion et pour assurer la bio-compatiblité.

Ce fil élastique est susceptible d'être conformé selon une géométrie désirée par traitement mécanique et thermique, selon des techniques connues en technologie de fils métalliques à caractéristiques de ressort à effet permanent.

Le diamètre du fil est de préférence compris entre 0,25mm et 1mm, typiquement de l'ordre de 0,37mm pour pouvoir être introduit dans un cathéter 6F. La grandeur F, French, vaut 0,33mm. Le cathéter 6F a donc un diamètre extérieur de 1,98mm et un calibre utile intérieur de l'ordre de 1,45mm de diamètre. Le choix d'un cathéter 6F est impossible pour la mise en place des filtres traditionnels effectivement utilisés et qui ont un diamètre trop gros et ne peuvent donc être mis en place que par le canal des veines de fortes sections, telles que les veines fémorales ou jugulaires. Par contre, dans le cadre de l'invention, on réalise des filtres susceptibles de circuler à l'intérieur d'un cathéter 6F de petit diamètre, ce qui permet la mise en place de ces filtres par le canal des petites veines. On partira par exemple d'une veine du pli du coude.

La longueur du fil constituant le filtre est de préférence de 22 à 35cm selon la taille du filtre adaptée, comme on le verra ci-après, au calibre de la veine cave inférieure du patient.

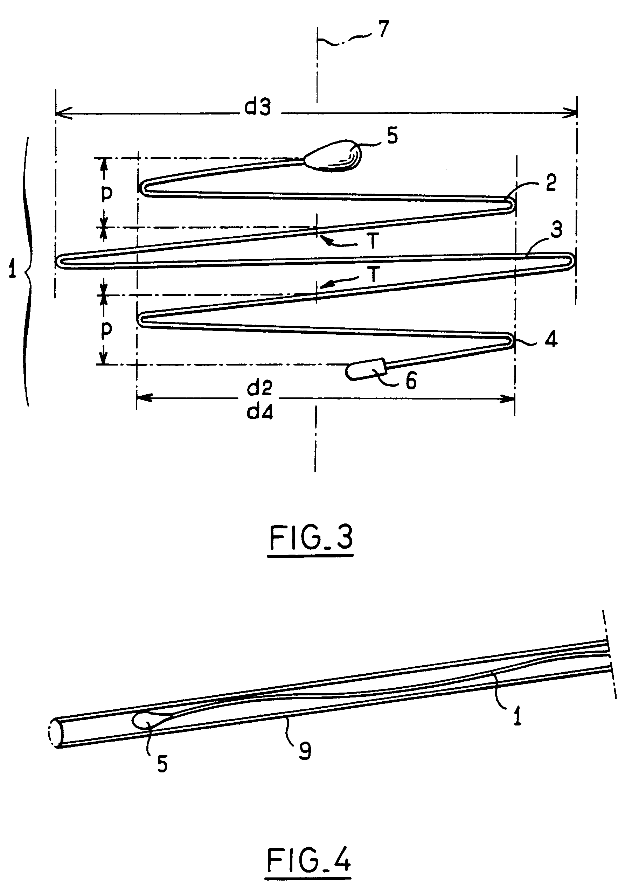

La qualité élastique du fil utilisé et son faible diamètre lui permettent, une fois qu'il est rendu quasi-rectiligne sous l'effet d'une faible traction, d'être introduit à l'intérieur d'un cathéter de petit diamètre pour y être véhiculé aussi bien dans un sens que dans l'autre sans contrainte, comme le représente la figure 4.

### 2. CONFORMATION DU FILTRE ET DE SES EXTREMITES ET LEURS FONCTIONS.

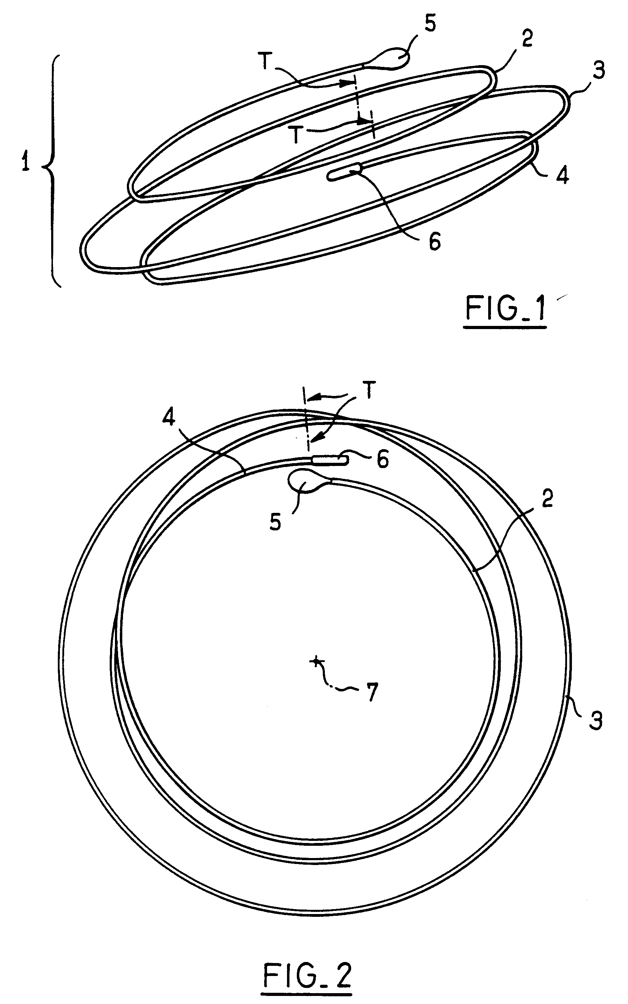

On se reportera aux figures 1, 2 et 3.

Le filtre 1 est conformé, selon la caractéristique principale de l'invention, en une spirale à trois spires 2, 3 et 4 non jointives, la spire médiane 3 ayant un diamètre d3 supérieur aux diamètres d2 et d4 des deux autres spires 2, 4. Sur les figures 1, 2 et 3, on a marqué par deux petits traits transversaux référencés T, les bornes séparant les spires 2, 3 et 4 pour bien montrer la géométrie du filtre.

Comme on le voit bien sur les figures 2 et 3, les spires 2, 3 et 4 sont à peu près coaxiales et les diamètres d2 et d4 sont par exemple égaux, sans que cette dernière condition soit critique. Les spires 2, 3 et 4 sont sensiblement centrées sur un axe commun 7 normal au plain de la figure 2. Ce qui est primordial, c'est que le diamètre d3 de la spire médiane 3 soit de valeur supérieure aux diamètres d2 et d4 des spires d'extrémité 2 et 4.

De préférence le diamiètre d3 de la spire médiane 3 est ≧ 1,25 d2 ou d4, très avantageusement d3 ≧ 1,4 d2 ou d4. Les trois spires 2, 3 et 4 ont de préférence des pas p sensiblement identiques.

Le pas p, de la spirale, de la figure 3, est au minimum de l'ordre de 3mm au repos avant implantation de sorte que l'épaisseur axiale totale de la spirale est au minimum d'environ 9mm au repos, quelque soit la valeur du diamètre d3 de la plus grande spire médiane 3.

Selon une autre caractéristique avantageuse de la presente invention, le diamètre d3 de la spire médiane 3 est supérieur à deux fois, typiquement ≧ 2,5 fois l'épaisseur axiale 3p du filtre au repos, pour permettre une mise en place correct du filtre.

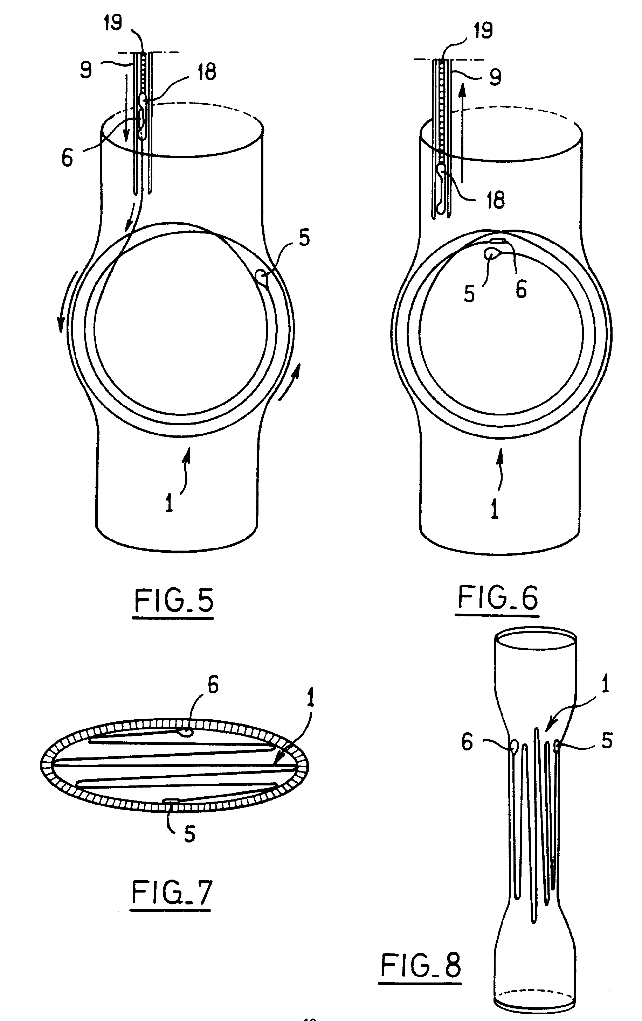

En ce qui concerne la taille du filtre 1, elle doit être adaptée à la veine cave à appareiller. La taille du filtre correspond au diamètre d3 de la plus grande spire médiane 3. Cette taille doit se rapprocher le plus possible de la valeur de la demi-circonférence de la veine cave inférieure calculée à partir de son diamètre mesuré comme précisée ci-après. Ainsi, la spire de diamètre d3 a toujours un diamètre plus grand que celui de la veine cave (figures 5, 6 et 7) pour obtenir le meilleur effet d'aplatissement de la veine cave par le filtre en place. En pratique on peut choisir une taille 1 de diamètre d3 de 27mm pour les veines caves de diamètre 16 à 18mm, une taille 2 de diamètre d3 de 31,5mm pour des diamètres de veines caves de 19 à 21mm et une taille 3 de diamètre d3 de 36mm pour des diamètres de veines caves supérieurs à 22mm.

Les extrémités du filtre, comme le fil, sont radio-opaques aux rayons X pour être bien visibles sous amplificateurs de luminance.

La première extrémité proximale 5, figures 1 à 4, a une forme ovoïde en goutte d'eau, arrondie vers son bout libre, et conique en son bout rattaché au fil du filtre. Cette forme lui permet de pouvoir se déplacer dans les deux sens à l'intérieur de la lumière du cathéter sans l'endommager et sans contrainte. Une fois sortie du cathéter, cette extrémité 5 est la première à être en contact avec la paroi lisse de la veine cave inférieure. Sa forme arrondie évite toute agression de la paroi veineuse et lui permet de glisser sur cette paroi pour que les boucles des spires 2, 3, 4 formant le filtre puissent s'enrouler sans contrainte (figures 5 et 6). Par ailleurs, la forme en goutte d'eau 5 permet de réintroduire sans difficulté le filtre à l'intérieur d'un cathéter porteur sans buter sur l'orifice d'entrée de ce cathéter lors d'une opération de retrait. A titre d'exemple non limitatif, les dimensions de cette extrémité proximale ovoïde 5 du filtre sont de l'ordre de 1,3mm pour son plus grand diamètre et de 0,6mm pour son diamètre de raccordement avec le fil formant le filtre 1. Sa longueur est d'environ 3,5mm.

La seconde extrémité distale 6 du filtre, figures 1 à 3 est formée d'un petit cylindre radio-opaque. A titre d'exemple non limitatif, le cylindre 6 a un diamètre externe de l'ordre de 0,6mm et une longueur de l'ordre de 4mm. Il est rattaché sur l'extrémité distale du fil du filtre. Son extrémité libre est légèrement bombée, lisse, pour ne pas endommager la paroi de la veine cave, une fois en place.

La forme du filtre avec ses extrémités détermine ses qualités : pour sa mise en place dans la veine cave, pour la stabilité et le maintien corrects en position, et pour ses fonctions de filtrage efficace de caillots venant de la circulation veineuse.

La première spire 2, figures 1 à 6, qui s'enroule la première dans la veine cave, lorsque le filtre est poussé hors du cathéter a un diamètre inférieur à celui de la spire médiane 3 pour obtenir aisément ce premier enroulement.

L'enroulement se poursuit par celui de la spire médiane 3. Son diamètre d3, plus grand que celui de la veine où il est implanté déforme la veine cave en l'aplatissant dans le sens de la hauteur, c'est-à-dire dans le sens de circulation du flux sanguin. Cet effet d'aplatissement a pour résultat une forte diminution de la section de passage du flux sanguin dans la veine cave, comme on le voit bien sur les figures 7 et 8.

La troisième spire 4 a un diamètre du même ordre que la première spire 2. Elle s'enroule en dernier et, à sa sortie du cathéter, libère son extrémité distale 6 automatiquement par effet ressort.

Le fait que la spirale, sur toute sa longueur présente une courbure sensiblement continue et égale garantit le maintien de sa conformation à l'entrée du cathéter, puis à sa sortie, malgré sa déformation transitoire rectiligne lors de son transfert par le cathéter. Par ailleurs, la forte pression exercée par les arcs de cercle des spires en contact avec la paroi veineuse, et essentiellement par ceux de la spire médiane, garantit le maintien en place du filtre sans aucun risque de migration.

L'épaisseur du filtre, dûe au pas p des trois spires, comme on le voit particulièrement sur les figures 7 et 8, permet d'arrêter et de garder des caillots ayant des plus petites dimensions de l'ordre de 3mm. Ils viennent s'entrelacer dans les boucles du filtre constituant une grille dans la lumière aplatie de la veine.

Le filtrage obtenu est dû à deux effets cumulés : l'aplatissement de la veine cave dans la zone d'implantation d'une part, la présence des différents arcs du filtre en travers de la veine cave d'autre part.

Le nombre des trois spires pour former la spirale s'est avéré optimum pour obtenir l'effet de filtrage. Un plus grand nombre de spires accroitrait inutilement la longueur du fil déroulé.

Ceci aurait pour conséquence une difficulté accrue de transfert à travers le cathéter.

Un nombre plus faible réduirait l'effet de filtrage.

Toutefois, la définition de l'invention ne doit pas être limitée rigoureusement à une géométrie à trois spires. Il est clair en effet qu'un filtre ayant un nombre de spires légèrement supérieur ou inférieur à trois donnerait satisfaction.

La conformation, non traumatisante pour la veine, du filtre, évite toute pénétration dans la paroi veineuse de quelque partie de ce filtre. Physiologiquement après implantation, les arcs des spires en contact avec la paroi veineuse sont recouverts progressivement de tissu endothélial. Le temps de fixation définitif du filtre dans la veine est au moins de l'ordre de 14 jours de sorte que, pendant ce temps, il est toujours possible de procéder à son retrait percutané, c'est-à-dire sans opération chirurgicale.

### 3. KITS DE STOCKAGE, PRESENTATION ET UTILISATION D'UN FILTRE.

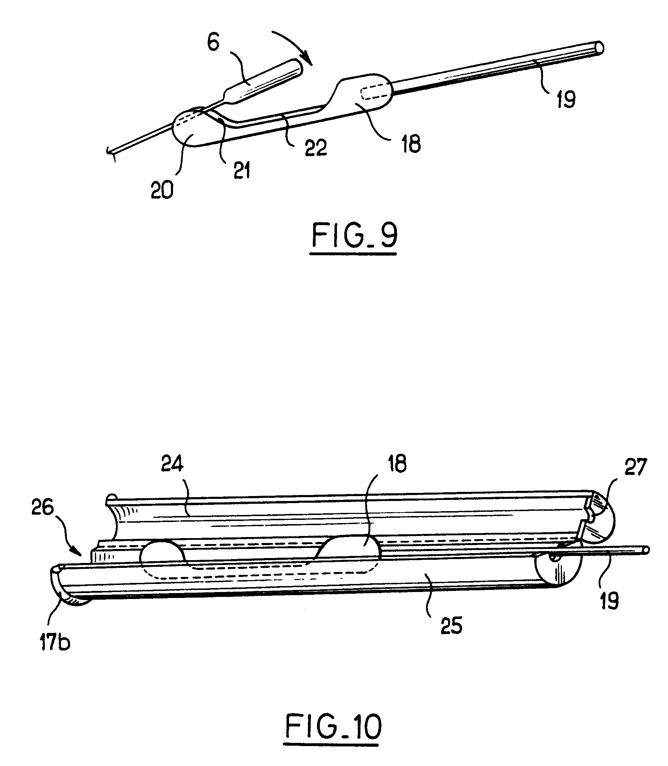

On se reportera maintenant aux figures 9 à 11 pour décrire une première variante de réalisation d'un kit adapté au filtre de l'invention pour en assurer optimalement le stockage, la présentation, et la mise en oeuvre en milieu médical.

Le kit comprend en premier lieu un cathéter 9, choisi de préférence de calibre 6F pour les raisons exposées ci-dessus. Il est réalisé, par exemple, en polyéthylène souple et radio-opaque. Sa longueur doit être au moins d'un mètre.

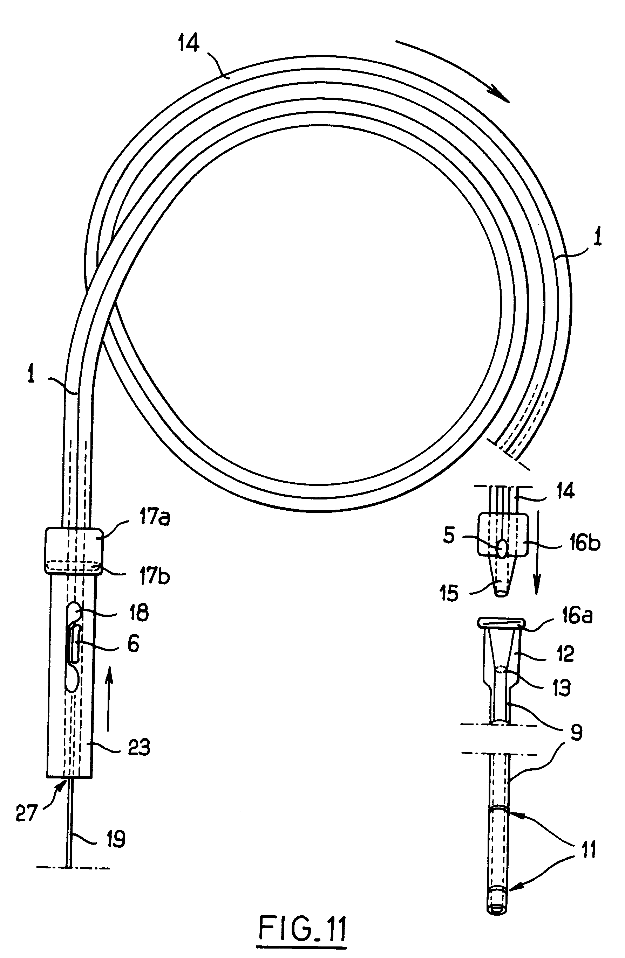

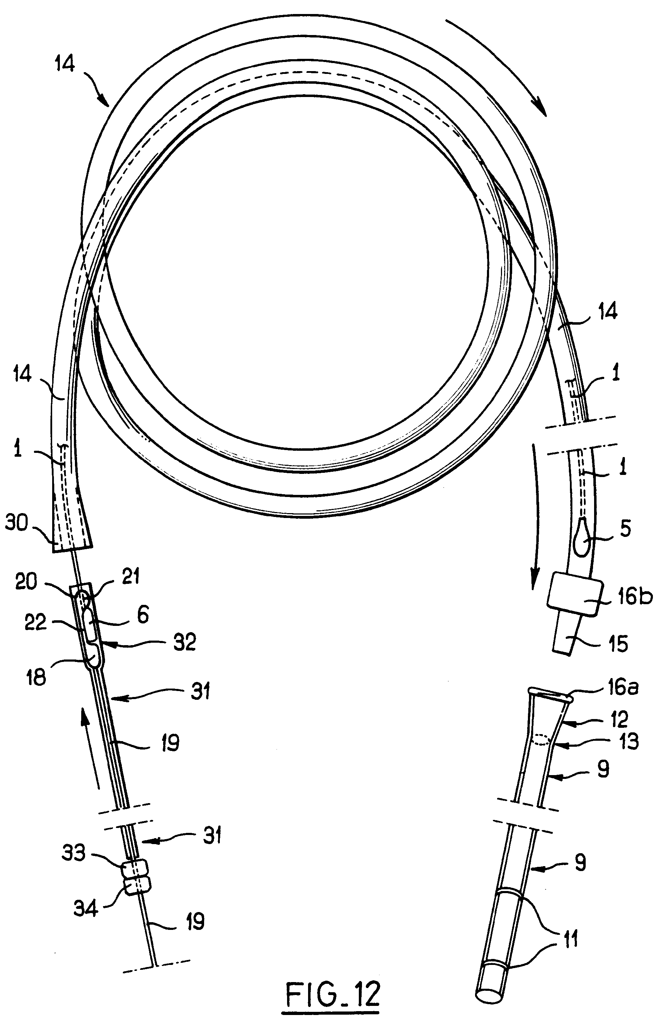

Son extrémité proximale, la première introduite dans la veine, présente deux bagues fines 11 fixées à l'extérieur, plus radio-opaques que le cathéter lui-même, distantes de 20mm (figures 11 et 12). Leur rôle est de permettre une mesure du coefficient d'agrandissement nécessaire pour corriger les distances modifiées par la distorsion dûe aux rayons X. On peut ainsi déterminer la taille 1, 2 ou 3 (voir ci-dessus) du filtre adapté au diamètre mesuré de la veine cave du patient traité.

L'extrémité distale de ce cathéter comporte un embout rigide 12 à pas de vis 16a, aux normes internationales. Son orifice est conique, large à son entrée. Il prend le diamètre de la lumière du cathéter quand il le rejoint par sa section 13.

Le kit comprend en second lieu un tube élastique transparent 14, de même configuration en spirale à spires non jointives que le filtre 1, d'une longueur au moins égale à celle du filtre, et lui servant de conteneur de stockage, de présentation et de mise en oeuvre.

Dans l'emballage du kit, avant utilisation, le cathéter 9 est à part, ainsi que les autres éléments du kit. Le tube conteneur 14 contient le filtre 1, l'extrémité ovoïde proximale 5 étant à l'intérieur du conteneur 14 et le petit cylindre distal 6 dépassant de l'extrémité distale du tube conteneur 14. Le filtre 1 est ainsi stocké sous sa forme de spirale à trois spires non jointives.

Le calibre intérieur du tube 14 est exactement le même que celui du cathéter 6F, soit 1,45mm, afin que le filtre, par son extrémité 5 et un poussoir à embout décrit ci-après, puissent y progresser sans contrainte.

La première extrémité proximale du tube 14 est solidarisée à l'extrémité distale du cathéter 9 par une vis mobile 16b située avant l'extrémité cylindro-conique du tube 14. Cette vis 16b vient se visser au pas de vis de l'embout rigide 12 du cathéter 9.

A l'autre extrémité du tube 14, il y a une seconde vis 17a mobile sur son axe.

Le kit comprend en troisième lieu un poussoir représenté sur la figure 9. Ce poussoir est constitué d'un embout rigide 18 auquel est soudé de façon coaxiale un cable d'acier souple tressé 19 par exemple de diamètre de 0,7mm d'une longueur correspondant au moins à la somme de la longueur du cathéter et de celle du filtre.

L'embout rigide 18, typiquement de longueur 10 à 12mm, rectiligne, cylindrique et de diamètre de 1,3mm, adapté au calibre interne du cathéter 6F 9 pour pouvoir y glisser sans frottement, a une extrémité libre 20 mousse, arrondie, hémisphérique, non traumatique pour le cathéter et la veine. Cette extrémité arrondie comporte une première encoche latérale 21 dans le sens de la longueur faite pour recevoir le fil du filtre 1. A cette encoche 21 fait suite un évidement 22 de longueur 5 à 6mm fait pour recevoir le petit cylindre distal 6 du filtre.

Le kit comprend enfin, en quatrième lieu, un boîtier d'assemblage du filtre avec le poussoir, représenté sur la figure 10.

Ce boîtier est consitué d'un tube plastique rigide cylindrique fait de deux hémicylindres 24 et 25 de même diamètre. Les hémicylindres 24, 25 sont unis par une charnière souple, mobile, de plastique. Initialement, ce tube rigide reste en position ouverte. Sa longueur est de l'ordre de 30 à 35mm. Son diamètre externe est fait pour qu'un pas de vis 17b situé à sa première extrémité 26 puisse venir se visser sur la vis mobile 17a, (figure 11), quand le boîtier est fermé. Dans cet état, les deux orifices extrêmes, de la face proximale 26, et de la face distale 27 sont de calibres différents.

Le premier orifice proximal 26, comportant en périphérie le pas de vis 17b, a un diamètre exactement identique au calibre interne du tube souple 14 et du cathéter 6F, soit 1,45mm.

Par cet orifice 26, l'embout 18 du poussoir contenant le cylindre 6 distal du filtre 1, figure 11, peut se glisser dans le tube 14 lorsque le boîtier 23 est vissé sur ce dernier.

Le second orifice distal 27 figures 10 et 11, est de calibre un peu supérieur à celui du câble 19 du poussoir 18, mais il est intérieur à celui de l'orifice 26 pour éviter que l'embout 18 du poussoir puisse s'échapper du boîtier rigide 23 quand il est fermé.

Les deux hémicylindres 24 et 25 sont identiques. En position fermée, le diamètre intérieur est le même que celui du tube 14, soit 1,45mm. Le rôle de ce boîtier rigide est de pouvoir solidariser facilement l'extrémité 6 du filtre 1 à l'évidement 22 du poussoir 18. Quand le cylindre 6 est posé dans l'évidement 22, il suffit de fermer le boîtier rigide 23, puis de le visser par sa première extrémité à pas de vis 17b sur le raccord 17a du tube 14 pour que le filtre, par l'intermédiaire du cylindre 6, soit rendu solidaire du poussoir 18. Ensuite, on peut amener par simple poussée sur le câble 19 le filtre 1 jusqu'à l'extrémité proximale du cathéter 9 et le larguer dans la veine cave. Quand les trois spires circulaires du filtre 1 ont repris leur forme dans la veine cave, on libère l'extrémité distale 6 en poussant dans la lumière veineuse l'embout du poussoir 18. Automatiquement, par effet ressort, l'extrémité 6 quitte le logement 22 de l'embout 18. Mais, si une difficulté de largage du filtre se présente, il est alors toujours possible de retirer l'ensemble du filtre 1 et du poussoir 18, en tirant sur le câble 19 du poussoir. A la fin du retrait, il suffit de dévisser le boîtier rigide 23 du tube 14. Le boîtier s'ouvre alors automatiquement et l'extrémité 6 du filtre sort automatiquement par son effet de ressort du logement 22 du poussoir 18.

Une seconde variante de réalisation de kit est représentée sur la figure 12. Sur cette figure, les mêmes numéros de référence que ceux de la figure 11 repèrent des éléments identiques. On retrouve sur la figure 12, un cathéter 9, un tube 14, le filtre 1 et un poussoir 18 équipé d'un câble 19.

L'extrémité distale du tube 14 est conformée en embout cônique femelle 30.

Le kit de la figure 12 ne comprend plus de boîtier 23, mais comprend une gaine comportant une première partie tubulaire souple 31, de calibre interne et de longueur choisis tels que le câble de poussoir 19 puisse y être guidé et y glisser librement.

La gaine a un diamètre externe inférieur ou égal au calibre du tube 14 et du cathéter 9.

La gaine comprend une seconde partie proximale rigide en forme de cloche tubulaire cylindrique 32. Cette cloche reçoit l'extrémité distale du filtre 6 logée manuellement dans le logement 22 du poussoir à embout rigide 18. Lorsque ceci est obtenu, on interdit un mouvement de retrait de la gaine 31 sur le câble 19 du poussoir par un moyen d'écrou fendu 33 et de contre-écrou 34 se solidarisant avec le câble 19 et placés côté extrémité distale de la gaine pour servir de butée à celle-ci.

Ensuite, au moment de la mise en oeuvre du filtre, il est très aisé d'introduire la cloche 32 dans l'extrémité femelle 30 du tube transparent.

Pour commander le largage de l'extrémité 6 du filtre 1, il faut désolidariser au moment voulu la pièce 18 du poussoir et l'extrémité élastique à effet ressort 6 du filtre. Dans le flux veineux, en dehors de la lumière du cathéter, la cloche 32 de l'extrémité proximale de la gaine souple 31 recouvre le poussoir 18 et l'extrémité 6 solidaires. Après desserrage des écrous 33 et 34, la simple traction de cette gaine souple sur le poussoir permet de découvrir la pièce 18 de laquelle sort automatiquement l'extrémité 6 du filtre. Ainsi, on peut contrôler la bonne reprise en forme de l'ensemble des trois spires de la spirale formant le filtre 1 avant de larguer sa dernière extrémité.

### 4. MOYENS DE RETRAIT DU FILTRE.

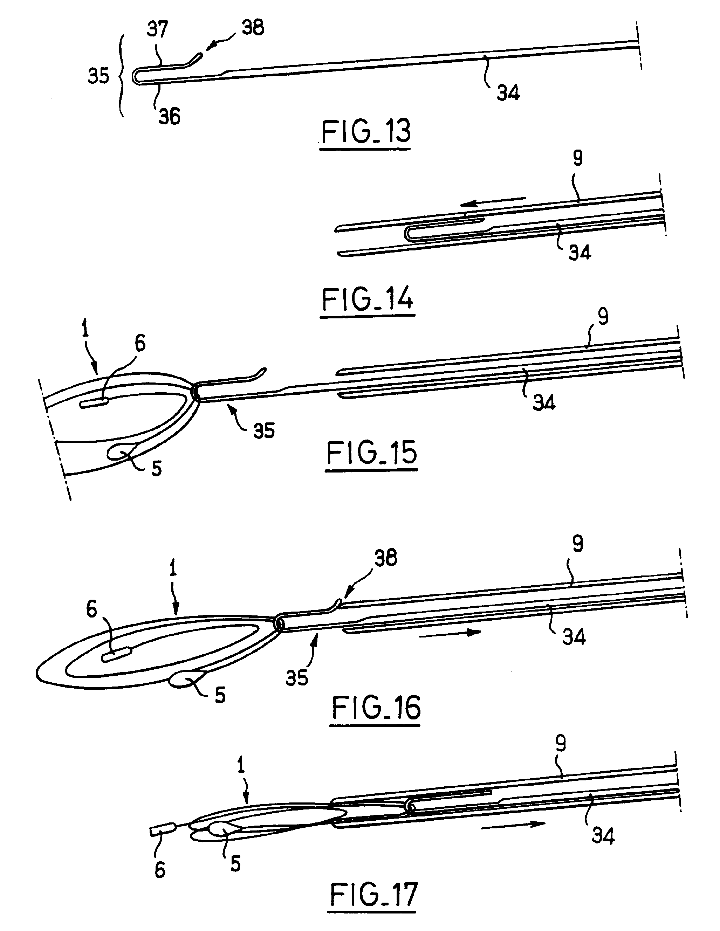

On procèdera maintenant à la description d'un guide à crochet permettant à tout moment de procéder à la récupération du filtre. Ce guide à crochet est représenté sur les figures 13 à 18. Il est associé à un cathéter 9 similaire à celui précédemment décrit. Le guide 34 est souple et peut prendre toutes les courbures imposées par le cathéter, sans difficulté de progression dans celui-ci. Le guide est en alliage d'acier, non thrombogène, biocompatible, recouvert de polytétrafluoroéthylène, connu commercialement sous la marque Téflon. Il est opaque aux rayons X. On rappelle que le diamètre interne du cathéter 6F est de 1,45mm.

Le crochet 35 situé à la tête de ce guide, est dans l'axe de celui-ci. Sa courbure extrême lui donne une forme en épingle à cheveux. Le crochet 35 est rigide. Il est radio-opaque, en acier. Son diamètre est inférieur à celui du guide lui-même, par exemple de 0,4mm, et sa longueur de l'ordre de 12mm. Ainsi, la boucle formée laisse un plus grand espace entre les deux branches 36 et 37 formant le crochet 35.

L'extrémité libre 38 de la branche 37 du crochet 35 fait un angle de 30° à 45° vers l'extérieur. Cette extrémité souple flagellée mesure de 2 à 3mm. Elle a la qualité d'être élastique. Ainsi, le crochet est non traumatisant pour la paroi de la veine cave inférieure et pour le cathéter porteur. De plus, son ouverture vers l'extérieur facilite les possibilités de crochetage d'une ou de plusieurs spires du filtre en place dans la veine cave inférieure comme représenté sur la figure 15.

Une fois le filtre attrapé par le crochet 35 du guide de récupération 34, on peut retirer celui-ci dans la lumière du cathéter 9, comme illustré sur la figure 17.

L'extrémité souple 38 qui termine le crochet permet de le fermer, soit en restant juste sur l'orifice proximal du cathéter 9 figure 16, soit en entrant dans celui-ci, figure 17.

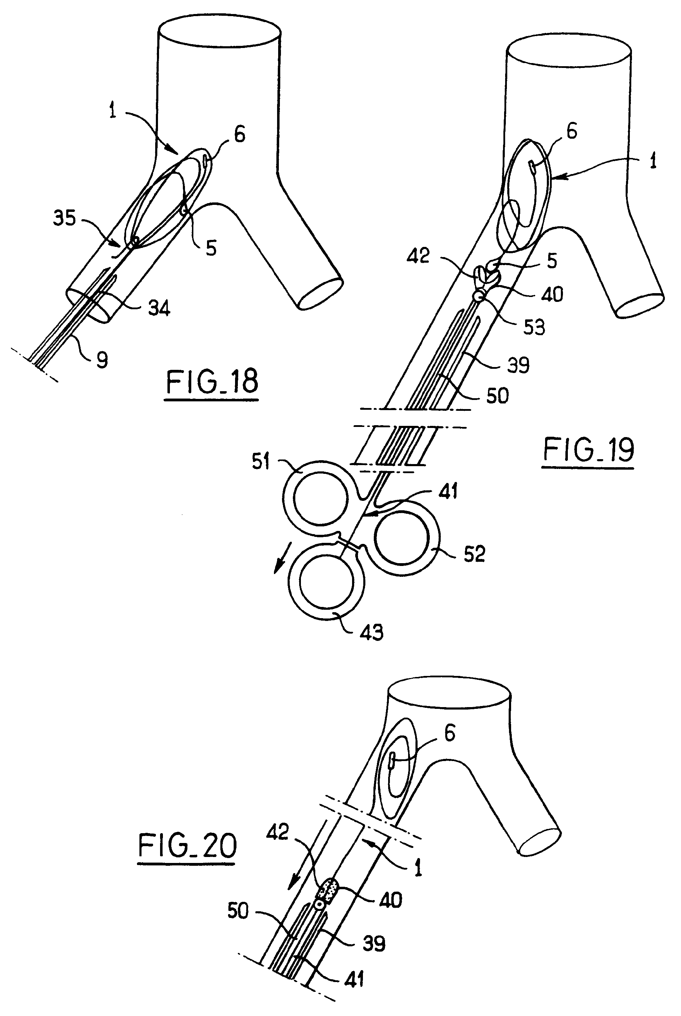

La ou les spires crochetées ne peuvent plus s'échapper du crochet 35. L'ensemble du filtre progresse dans la lumière de la veine cave inférieure par traction, figure 18. La souplesse du filtre et son élasticité lui permettent de se plier pour être totalement ou en partie rentré dans le cathéter 9.

L'ensemble formé par le cathéter porteur 9, le guide 34 de récupération à crochet 35 et le filtre 1 est retiré jusqu'à l'orifice d'entrée de la veine dans laquelle ces éléments avaient été introduits, le filtre replié sur lui-même prenant le diamètre interne de la veine dans laquelle il progresse. Cette manipulation de récupération du filtre par crochetage peut être effectuée dans n'importe quel sens de progression du cathéter 6F dans la veine cave. Ainsi, l'abord peut être fait par la veine fémorale ou la veine jugulaire interne du cou.

La manoeuvre de retrait peut également s'effectuer à l'aide d'un outil plus élaboré représenté sur les figures 19 et 20. Cet outil dit pince à retrait comporte essentiellement un corps support allongé 50, susceptible d'être engagé dans un cathéter 39 et muni de deux anneaux de préhension 51, 52 à son extrémité distale et deux mâchoires 40, 42 radio-opaques arrondies et creuses susceptibles d'être ouvertes ou fermées, qui sont articulées sur l'extrémité proximale du support 50, autour d'un axe 53. Les mâchoires 40, 42 sont de préférence sollicitées élastiquement à l'ouverture.

Elles peuvent être commandées à la fermeture par traction (ou poussée) sur un câble 41 qui chemine le long du support 50 et aboutit à un anneau de traction 43 accessible à l'extrémité distale du support 50. L'organe élastique sollicitant les mâchoires à l'ouverture n'est pas représenté sur les figures 19 et 20 pour simplifier l'illustration.

Ouverte, la pince peut attraper l'une des deux extrémités 5 ou 6 du filtre, figure 19. La pince est ensuite refermée, par commande du câble 41, et par simple traction le filtre se déroule dans la veine et suit le trajet de la pince que l'on retire à travers le cathéter 39 rigide comme schématisé sur la figure 20.

La longueur de la pince de retrait est variable, de 50cm à 1m. C'est un outil souple de diamètre externe adapté pour progresser à l'intérieur d'un cathéter porteur rigide 39 d'un calibre supérieur à celui de mise en place du filtre. Ce cathéter 39 est lui aussi introduit de façon percutanée.

L'ensemble de l'outil est conformé de manière à être non traumatisant. Les mâchoires ne peuvent endommager la paroi veineuse.

La manoeuvre de retrait peut être effectué à partir de la veine jugulaire ou fémorale.

Bien entendu, la description précédente de réalisations particulières de l'invention n'a été donnée qu'à titre d'exemple. Bien des variantes, de réalisation et/ou de dimensionnement, peuvent être imaginées sans pour autant sortir du cadre de l'invention comme défini dans les revendications suivantes.

## Revendications

1. Filtre anti-embolie pulmonaire, réalisé en un fil élastique à effet de ressort rémanent conformé en spirale, caractérisé en ce que la spirale (1) comprend trois spires non jointives (2, 3, 4) sensiblement circulaires dont la spire médiane (3) a un diamètre (d3) supérieur à ceux (d2, d4) des deux autres spires, que ce diamètre (d3) est choisi voisin de la valeur de la demi-circonférence de la veine cave dans la zone où le filtre doit être implanté pour assurer optimalement la fonction de maintien en place du filtre par aplatissement de la veine cave.

2. Filtre anti-embolie pulmonaire selon la revendication 1, caractérisé en ce que l'extrémité proximale (5) du filtre est munie d'un élément ovoïde radio-opaque.

3. Filtre anti-embolie pulmonaire selon l'une des revendications 1 et 2, caractérisé en ce que l'extrémité distale (6) du filtre est munie d'un petit cylindre radio-opaque à bout libre arrondi.

4. Filtre anti-embolie pulmonaire selon l'une des revendications 1 à 3, caractérisé en ce qu'il est réalisé en métal.

5. Filtre anti-embolie pulmonaire selon la revendication 4, caractérisé en ce qu'il est réalisé à base d'un alliage choisi dans le groupe comprenant les alliages suivants : 1) cuivre et nickel et aluminium, 2) cuivre et zinc et aluminium, 3) cuivre et zinc et aluminium et nickel, 4) cuivre et étain et nickel.

6. Filtre anti-embolie pulmonaire selon l'une des revendications 1 à 5, caractérisé en ce que l'ensemble du fil du filtre et de ses extrémités est revêtu d'une mince couche d'or.

7. Filtre anti-embolie pulmonaire selon l'une des revendications 1 à 6, caractérisé en ce que les deux spires latérales (2, 4) ont des diamètres (d2, d4) au moins sensiblement égaux.

8. Filtre anti-embolie pulmonaire selon l'une des revendications 1 à 7, caractérisé en ce que les trois spires (2, 3, 4) sont au moins sensiblement coaxiales.

9. Filtre anti-embolie pulmonaire selon l'une des revendications 1 à 8, caractérisé en ce que le diamètre du fil est compris entre 0,25 et 1 mm.

10. Filtre anti-embolie pulmonaire selon la revendication 9, caractérisé en ce que le diamètre du fil est de l'ordre de 0,37mm.

11. Filtre anti-embolie pulmonaire selon l'une des revendications 1 à 10, caractérisé en ce que la longueur totale du filtre est de l'ordre de 22 à 35cm.

12. Filtre anti-embolie pulmonaire selon l'une des revendications 1 à 11, caractérisé en ce que le diamètre (d3) de la spire médiane (3) est supérieur ou égal à 1,25 fois le diamètre (d2, d4) des spires latérales (2, 4).

13. Filtre anti-embolie pulmonaire selon l'une des revendications 1 à 12, caractérisé en ce que le diamètre (d3) de la spire médiane (3) est supérieur ou égal à 1,4 fois le diamètre (d2, d4) des spires latérales (2, 4).

14. Filtre anti-embolie pulmonaire selon l'une des revendications 1 à 13, caractérisé en ce que les pas des différentes spires (2, 3, 4) sont sensiblement égaux.

15. Filtre anti-embolie pulmonaire selon l'une des revendications 1 à 14, caractérisé en ce que le pas (p) de la spirale au repos, avant implantation, est d'au moins 3mm.

16. Filtre anti-embolie pulmonaire selon l'une des revendications 1 à 15, caractérisé en ce que l'épaisseur axiale du filtre au repos, avant implantation, est d'au moins 9mm.

17. Filtre anti-embolie pulmonaire selon l'une des revendications 1 à 16, caractérisé en ce que le diamètre (d3) de la spire médiane (3) est ≧ 2 fois, typiquement ≧ 2,5 fois l'épaisseur axiale au repos, avant implantation du filtre.

18. Filtre anti-embolie pulmonaire selon la revendication 2, caractérisé en ce que l'élément ovoïde (5) prévu à l'extrémité proximale du filtre a un diamètre maximum de l'ordre de 1,3mm et une longueur de l'ordre de 3,5mm.

19. Filtre anti-embolie pulmonaire selon la revendication 3, caractérisé en ce que l'élément cylindrique (6) prévu à l'extrémité distale du filtre a un diamètre de l'ordre de 0,6mm et une longueur de l'ordre de 4 mm.

20. Kit destiné au stockage, à la présentation et à la mise en place d'un filtre anti-embolie pulmonaire selon l'une des revendications 1 à 19, comprenant :
- un cathéter (9),
- un poussoir constitué d'un embout rigide (18) présentant, en son extrémité proximale arrondie, une encoche longitudinale (21) pour recevoir l'extrémité distale (4) du fil élastique du filtre, puis un logement (22) pour recevoir le petit cylindre distal (6) du filtre, l'embout rigide étant solidaire en son extrémité distale d'un câble (19), et
- des moyens de bridage (24, 25 ; 31, 32) comprenant un conteneur apte à recevoir le poussoir (18) muni de l'extrémité distale (6) du filtre, caractérisé par le fait que :
- le cathéter (9) est terminé en son extrémité distale par un embout (12) muni de moyens de raccordement rapide (16a),
- le câble (19) fixé à l'extrémité distale de l'embout rigide (18) a une longueur au moins égale à la somme de la longueur du cathéter et de celle du filtre, et
par le fait que le kit comprend en outre :
- un tube élastique transparent (14) de même configuration en spirale que le filtre (1), d'une longueur au moins égale à celle du filtre et lui servant de conteneur de stockage et de présentation, ce tube étant équipé en son extrémité proximale de premiers moyens de raccordement rapide (16b) destinés à coopérer avec ceux précités (16a) du cathéter.

21. Kit selon la revendication 20, caractérisé en ce que le cathéter (9) est un cathéter de diamètre externe (6F) 1,98mm.

22. Kit selon l'une des revendications 20 ou 21, caractérisé en ce que les moyens de bridage sont constitués d'un boîtier rigide, cylindrique (23) formé de deux hémicylindres identiques (24, 25) reliés par une charnière et destinés à recevoir l'assemblage de l'extrémité distale (6) du filtre en place dans le logement (22) de l'embout rigide, l'extrémité proximale (26) de ce boîtier étant munie de moyens de raccordement rapide (17b) coopérant avec des seconds moyens de raccordement rapide prévus à l'extrémité distale du tube.

23. Kit selon l'une des revendications 20 ou 21, caractérisé en ce que les moyens de bridage sont constitués d'une gaine comportant une première partie (31), tubulaire, souple, de calibre interne et de longueur adaptés pour que le câble du poussoir puisse y être guidé et y glisser librement, et une seconde partie rigide en forme de cloche tubulaire cylindrique (32) susceptible, d'une part, de recevoir l'extrémité distale du filtre (6) logée manuellement dans le poussoir à embout rigide (18) et, d'autre part, d'être engagée manuellement dans l'extrémité distale (30) du tube élastique transparent (14), le diamètre maximal extérieur de la gaine étant légèrement inférieur au diamètre interne du cathéter, et des moyens (33, 34) étant prévus pour pouvoir interdire sélectivement la sortie du poussoir de l'extrémité de la gaine.

24. Kit selon la revendication 23, caractérisé en ce que les moyens aptes à interdire sélectivement la sortie du poussoir hors de l'extrémité de la gaine comprennent un écrou et un contre-écrou serrés sur le câble (19) du poussoir (18).

25. Kit selon l'une des revendications 20 à 24, caractérisé en ce que l'extrémité proximale du cathéter (9) est munie de deux anneaux espacés (11) plus radio-opaques que le cathéter et permettant de mesurer le diamètre de la veine cave.

26. Kit selon l'une des revendications 20 à 25, caractérisé en ce qu'il comprend en outre un guide à crochet de récupération de filtre constitué d'un guide souple (34) pouvant coulisser librement dans un cathéter (9) et terminé par un crochet rigide (35) dans l'axe du guide et ayant une forme d'épingle à cheveux.

27. Kit selon la revendication 26, caractérisé en ce que la branche libre (37) du crochet rigide (35) se termine par une extrémité souple (38), inclinée vers l'extérieur du crochet pour avoir une ouverture élargie du crochet afin d'attraper le nombre maximal de spires du filtre à retirer.

28. Kit selon l'une des revendications 20 à 25, caractérisé en ce qu'il comporte une pince de retrait possédant deux mâchoires (40, 42) radio-opaques arrondies et creuses susceptibles d'être ouvertes ou fermées, articulées à une extrémité d'un corps support allongé (50).

## Claims

1. An anti-pulmonary embolism filter of the type comprising a remanent spring effect resilient wire in form of a spiral, the filter being characterized in that the spiral (1) comprises three non-touching substantially circular turns (2,3,4) with the middle turn (3) having a diameter (d3) greater than the diameters (d2,d4) of the other two turns,said diameter (d3) being selected to be close to one-half the circumference of the vena cava in the zone where the filter is to be implanted in order to ensure that the filter is optimally held in place by flattening of the vena cava.

2. An anti-pulmonary embolism filter according to claim 1, characterized in that the proximal end (5) of the filter is provided with a radio-opaque ovoid element.

3. An anti-pulmonary embolism filter according to claim 1 or 2, characterized in that the distal end (6) of the filter is provided with a small radio-opaque cylinder having a rounded free end.

4. An anti-pulmonary embolism filter according to any one of claims 1 to 3, characterized in that it is made of metal.

5. An anti-pulmonary embolism filter according to claim 4, characterized in that it is made from an alloy selected from the group comprising the following alloys: 1) copper, nickel, and aluminum; 2) copper, zinc, and aluminum; 3) copper, zinc, aluminum, and nickel; and 4) copper, tin, and nickel.

6. An anti-pulmonary embolism filter according to any one of claims 1 to 5, characterized in that the wire of the filter and its ends are covered overall with a thin layer of gold.

7. An anti-pulmonary embolism filter according to any one of claims 1 to 6, characterized in that the two end turns (2, 4) are of at least substantially equal diameters (d2, d4).

8. An anti-pulmonary embolism filter according to any one of claims 1 to 7, characterized in that the three turns (2, 3, 4) are at least substantially coaxial.

9. An anti-pulmonary embolism filter according to any one of claims 1 to 8, characterized in that the diameter of the wire lies in the range 0.25 mm to 1 mm.

10. An anti-pulmonary embolism filter according to claim 9, characterized in that the diameter of the filter is about 0.37 mm.

11. An anti-pulmonary embolism filter according to any one of claims 1 to 10, characterized in that the total length of the filter is about 22 cm to 35 cm.

12. An anti-pulmonary embolism filter according to any one of claims 1 to 11, characterized in that the diameter (d3) of the middle turn (3) is greater than or equal to 1.25 times the diameter (d2, d4) of the end turns (2, 4).

13. An anti-pulmonary embolism filter according to any one of claims 1 to 12, characterized in that the diameter (d3) of the middle turn (3) is greater than or equal to 1.4 times the diameter (d2, d4) of the end turns (2, 3, 4).

14. An anti-pulmonary embolism filter according to any one of claims 1 to 13, characterized in that the pitch of the various turns (2, 3, 4) are substantially equal.

15. An anti-pulmonary embolism filter according to any one of claims 1 to 14, characterized in that the pitch (p) of the spiral when at rest and prior to implantation is not less than 3 mm.

16. An anti-pulmonary embolism filter according to any one of claims 1 to 15, characterized in that the axial extent of the filter at rest prior to implantation is at least 9 mm.

17. An anti-pulmonary embolism filter according to any one of claims 1 to 16, characterized in that the diameter (d3) of the middle turn (3) is ≧ 2 times and typically ≧ 2.5 times the axial extent at rest of the filter prior to being implanted.

18. An anti-pulmonary embolism filter according to claim 2, characterized in that the ovoid element (5) provided at the proximal end of the filter has a minimum diameter of about 1.3 mm and a length of about 3.5 mm.

19. An anti-pulmonary embolism filter according to claim 3, characterized in that the cylindrical element (6) provided at the distal end of the filter has a diameter of about 0.6 mm and a length of about 4 mm.

20. A kit for storing, presenting, and installing an antipulmonary embolism filter according to any one of claims 1 to 19, characterized in that it comprises:
- a catheter (9)
- a pusher constituted by a rigid endpiece (18) having a longitudinal notch (21) at its rounded proximal end for receiving the distal end (4) of the resilient wire of the filter, followed by a housing (22) for receiving the small distal cylinder (6) of the filter, the rigid endpiece being secured at its distal end to a cable (19) and
- clamping means (24, 25; 31, 32) including a container suitable for receiving the pusher (18) when this one is provided at the distal end (6) of the filter,
characterized in that:
- the catheter (9) is terminated at its distal end by an endpiece (12) provided with rapid coupling means (16a),
- the cable (19) secured at the distal end of the rigid endpiece (18) has a length at least equal to the sum of the length of the catheter plus the length of the filter, and by the fact that the kit further comprises:
- a transparent resilient tube (14) having the same spiral configuration as the filter (1), the length of the tube being not less than the length of the filter and the tube serving as a storage and presentation container for the filter, said tube being fitted at its proximal end with first rapid coupling means (16b) for co-operating with the above mentioned rapid coupling means (16a) of the catheter.

21. A kit according to claim 20, characterized in that the catheter (9) is a 6F catheter having an outside diameter of 1.98 mm.

22. A kit according to claim 20 or 21, characterized in that the clamping means comprise a rigid cylindrical housing (23) constituted by two identical half-cylinders (24, 25) connected together by means of a hinge and intended to receive the assembly of the distal end (6) of the filter as received in the housing (22) of the rigid endpiece, the proximal end (26) of said housing being provided with rapid coupling means (17b) cooperating with second rapid coupling means provided at the distal end of the tube.

23. A kit according to claim 20 or 21, characterized in that the clamping means are constituted by a sheath including a flexible tubular first portion (31) of internal caliber and of length adapted to enable the pusher cable to be guided therein and to slide freely therealong, and a rigid second portion in the form of a cylindrical tubular bell (32) suitable firstly for receiving the distal end of the filter (6) manually disposed in the pusher having the rigid endpiece (18), and secondly for being manually engaged in the distal end (30) of the transparent resilient tube (14), the maximum outside diameter of the sheath being slightly less than the inside diameter of the catheter, and means (33, 34) being provided to selectively prevent the pusher from leaving the end of the sheath.

24. A kit according to claim 23, characterized in that the means suitable for selectively preventing the pusher from leaving the end of the sheath comprise a nut and a lock nut clamped on the cable (19) of the sheath (18).

25. A kit according to any one of claims 20 to 24, characterized in that the proximal end of the catheter (9) is provided with two spaced-apart rings (11) that are more radio-opaque than the catheter and that enable the diameter of the vena cava to be measured.

26. A kit according to any one of claims 20 to 25, characterized in that it further includes a hook guide for recovering the filter, the guide being constituted by a flexible guide (34) capable of sliding freely inside a catheter (9) and terminated by a rigid hook (35) on the axis of the guide and in the form of a hairpin.

27. A kit according to claim 26, characterized in that the free branch (37) of the rigid hook (35) is terminated by a flexible end (38) sloping outwards from the hook so as to provide a flared opening to the hook for the purpose of catching the maximum number of turns of the filter to be withdrawn.

28. A kit according to any one of claims 20 to 25, characterized in that it includes a withdrawal grab having two jaws (40, 42) that are radio-opaque, rounded, and hollow, and that are suitable for being opened and closed, the jaws being hinged to one end of an elongate support body (50).

## Patentansprüche

1. Antiemboliefilter aus einem elastischen Draht in form einer Spirale mit der Wirkung einer remanenten Feder,
**dadurch gekennzeichnet,** **daß**
die Spirale (1) drei weitgehend kreisförmige nicht verbundene Windungen (2, 3, 4) aufweist, deren mittlere Windung (3) einen Durchmesser (3d) hat, der größer ist, als der Durchmesser (d2, d4) der beiden anderen Windungen, und daß der Durchmesser (d3) annähernd dem Wert des halben Kreisumfangs der Venenkaverne in dem Bereich gewählt wird, in dem der Filter implantiert werden soll, um die Halterungsfunktion des Filters an Ort und Stelle optimal durch Abflachung der Venenkaverne zu gewährleisten.

2. Antiemboliefilter nach Anspruch 1,
**dadurch gekennzeichnet,** **daß**
das nahe Ende (5) des Filters mit einem strahlenundurchlässigen ovalen Element versehen ist.

3. Antiemboliefilter nach einem der Ansprüche 1 oder 2,
**dadurch gekennzeichnet,** **daß**
das entfernte Ende (6) des Filters mit einem kleinen strahlenundurchlässigen Zylinder mit einem freien abgerundeten Ende versehen ist.

4. Antiemboliefilter nach einem der Ansprüche 1 bis 3,
**dadurch gekennzeichnet,** **daß**
er aus einem Metall hergestellt ist.

5. Antiemboliefilter nach Anspruch 4,
**dadurch gekennzeichnet,** **daß**
er aus einer Legierung besteht, die aus der Gruppe ausgewählt ist, die folgende Legierungen umfaßt: 1) Kupfer und Nickel und Aluminium, 2) Kupfer und Zink und Aluminium, 3) Kupfer und Zink und Aluminium und Nickel, 4) Kupfer und Zinn und Nickel.

6. Antiemboliefilter nach einem der Ansprüche 1 bis 5,
**dadurch gekennzeichnet,** **daß**
der gesamte Draht des Filters und seine Enden mit einer dünnen Schicht aus Gold überzogen sind.

7. Antiemboliefilter nach einem der Ansprüche 1 bis 6,
**dadurch gekennzeichnet,** **daß**
die beiden seitlichen Windungen (2, 4) mindestens weitgehend gleiche Durchmesser (d2, d4) haben.

8. Antiemboliefilter nach einem der Ansprüche 1 bis 7,
**dadurch gekennzeichnet,** **daß**
die drei Windungen (2, 3, 4) mindestens weitgehend koaxial sind.

9. Antiemboliefilter nach einem der Ansprüche 1 bis 8,
**dadurch gekennzeichnet,** **daß**
der Durchmesser des Drahtes zwischen 0,25 mm und 1 mm beträgt.

10. Antiemboliefilter nach Anspruch 9,
**dadurch gekennzeichnet,** **daß**
der Durchmesser des Drahtes eine Größenordnung von 0,37 mm hat.

11. Antiemboliefilter nach einem der Ansprüche 1 bis 10,
**dadurch gekennzeichnet,** **daß**
die Gesamtlänge des Filters innerhalb der Größenordnung von 22 cm bis 35 cm liegt.

12. Antiemboliefilter nach einem der Ansprüche 1 bis 11,
**dadurch gekennzeichnet,** **daß**
der Durchmesser (d3) der mittleren Windung (3) mindestens 1,25 mal den Wert des Durchmessers (d2, d4) der seitlichen Windungen (2, 4) hat.

13. Antiemboliefilter nach einem der Ansprüche 1 bis 12,
**dadurch gekennzeichnet,** **daß**
der Durchmesser (d3) der mittleren Windung (3) mindestens 1,4 mal den Wert des Durchmessers (d2, d4) der seitlichen Windungen (2, 4) hat.

14. Antiemboliefilter nach einem der Ansprüche 1 bis 13,
**dadurch gekennzeichnet,** **daß**
die Gewindesteigungen der verschiedenen Windungen (2, 3, 4) weitgehend gleich sind.

15. Antiemboliefilter nach einem der Ansprüche 1 bis 14,
**dadurch gekennzeichnet,** **daß**
vor der Implantation die Gewindesteigung (p) der Windung in Ruhestellung mindestens 3 mm beträgt.

16. Antiemboliefilter nach einem der Ansprüche 1 bis 15,
**dadurch gekennzeichnet,** **daß**
vor der Implantation die axiale Dicke des Filter in Ruhestellung mindestens 9 mm beträgt.

17. Antiemboliefilter nach einem der Ansprüche 1 bis 16,
**dadurch gekennzeichnet,** **daß**
der Durchmesser (d3) der mittleren Windung (3) ≧ 2 mal, typischerweise ≧ 2,5 mal den Wert der axialen Dicke in Ruhestellung vor der Implantation des Filters hat.

18. Antiemboliefilter nach Anspruch 2,
**dadurch gekennzeichnet,** **daß**
das am nahen Ende des Filters vorgesehene ovale Element (5) einen maximalen Durchmesser in der Größenordnung von 1,3 mm und eine Länge in der Größenordnung von 3,5 mm hat.

19. Antiemboliefilter nach Anspruch 3,
**dadurch gekennzeichnet,** **daß**
das am entfernten Ende des Filters vorgesehene zylindrische Element (6) einen Durchmesser in der Größenordnung von 0,6 mm und eine Länge in der Größenordnung von 4 mm hat.

20. Kit für die Aufbewahrung, Verpackung und Anwendung eines Antiemboliefilters nach einem der Ansprüche 1 bis 19, der folgendes aufweist:
- einen Katheter (9),
- einen aus einem steifen Ansatz gebildeten Stößel (18), der an seinem abgerundeten nahen Ende eine längliche Einkerbung (21) für die Aufnahme des entfernten Endes (4) des elastischen Drahtes des Filters aufweist, sowie eine Lagerung (22) für die Aufnahme des kleinen entfernten Zylinders (6) des Filters, wobei dieser steife Ansatz an seinem entfernten Ende mit einem Kabel (19) fest verbunden ist, sowie
- Flanschmittel (24, 25; 31, 32) mit einem Behälter für die Aufnahme des mit dem entfernten Ende (6) des Filters versehenen Stößels (18),
**dadurch gekennzeichnet,** **daß**
- der Katheter (9) an seinem entfernten Ende einen mit einem Schnellverschluß (16a) versehenen Ansatz (12) aufweist,
- das an dem entfernten Ende des steifen Ansatzes (18) befestigte Kabel (19) eine Länge hat, die mindestens gleich der Summe aus der Länge des Katheters und aus der Länge des Filters ist, und
dadurch daß der Kit weiterhin folgendes aufweist:
- einen transparenten elastischen Schlauch (14) gleicher spiralenförmiger Konfiguration wie der Filter (1) mit einer Länge aufweist, die mindestens gleich der Länge des Filters ist und als Lagerungs- und Verpackungsbehälter dient, wobei dieser Schlauch an seinem nahen Ende mit ersten Mitteln für einen Schnellverschluß (16b) ausgerüstet ist, die mit den vorgenannten Schnellverschlußmitteln (16a) des Katheters zusammenwirken.

21. Kit nach Anspruch 20,
**dadurch gekennzeichnet,** **daß**
der Katheter (9) ein Katheter mit einem Außendurchmesser (6F) von 1,98 mm ist.

22. Kit nach einem der Ansprüche 20 oder 21,
**dadurch gekennzeichnet,** **daß**
die Flanschmittel aus einem steifen zylindrischen Gehäuse (23) bestehen, das aus zwei identischen Halbzylindern (24, 25) besteht, die durch ein Gelenk verbunden und dafür bestimmt sind, den Zusammenbau des entfernten Endes (6) des in der Aufnahme (22) des steifen Ansatzes sitzenden Filters aufzunehmen, wobei das nahe Ende (26) dieses Gehäuses mit Schnellverschlußmitteln (17b) ausgerüstet ist, die mit zweiten Schnellverschlußmitteln zusammenwirken, die am entfernten Ende des Schlauches vorgesehen sind.

23. Kit nach einem der Ansprüche 20 oder 21,
**dadurch gekennzeichnet,** **daß**
die Flanschmittel aus einem Hüllrohr bestehen, das einen ersten elastischen rohrförmigen Teil (31) mit einer Innenabmessung und einer Länge aufweist, die so ausgelegt sind, daß das Kabel des Stößels darin geführt werden und frei gleiten kann, sowie einen zweiten steifen Teil in form einer rohrförmigen zylindrischen Glocke (32) aufweist, der einerseits das entfernte Ende des Filters (6) aufnehmen kann, das von Hand in den aus einem steifen Ansatz bestehenden Stößel (18) eingelegt wird, und andererseits von Hand in das entfernte Ende (30) des transparenten elastischen Schlauches (14) eingesetzt werden kann, wobei der maximale Außendurchmesser des Hüllrohres geringfügig kleiner ist, als der Innendurchmesser des Katheters, und daß Mittel (33, 34) vorgesehen sind, um wahlweise den Austritt des Stößels aus dem Ende des Hüllrohres zu verhindern.

24. Kit nach Anspruch 23,
**dadurch gekennzeichnet,** **daß**
die Mittel für die wahlweise Unterbindung des Austritts des Stößels aus dem Ende des Hüllrohres eine Mutter und eine Gegenmutter aufweisen, die auf das Kabel (19) des Stößels (18) aufgeschraubt sind.

25. Kit nach einem der Ansprüche 20 bis 24,
**dadurch gekennzeichnet,** **daß**
das nahe Ende des Katheters (9) mit zwei im Abstand angeordneten Ringen (11) ausgestattet ist, die stärker strahlenundurchlässig sind, als der Katheter und die es ermöglichen, den Durchmesser der Venenkaverne zu messen.

26. Kit nach einem der Ansprüche 20 bis 25,
**dadurch gekennzeichnet,** **daß**
er außerdem eine hakenförmige Führung- für die Rückholung des Filters aufweist, die aus einer elastischen Führung (34) besteht, die frei in einem Katheter (9) gleiten kann und in einem steifen Haken (35) in der Achse der Führung endet, der die form einer Haarnadel hat.

27. Kit nach Anspruch 26,
**dadurch gekennzeichnet,** **daß**
der freie Arm (37) des steifen Hakens (35) ein elastisches Ende (38) aufweist, das zur Außenseite des Hakens geneigt ist, um eine erweiterte Öffnung des Hakens zu bilden, um die maximale Anzahl von Windungen des herauszuziehenden Filters zu erfassen.

28. Kit nach einem der Ansprüche 20 bis 25,
**dadurch gekennzeichnet,** **daß**
er eine Ausziehzange mit zwei abgerundeten und hohlen strahlenundurchlässigen Klemmbacken (40, 42) aufweist, die geöffnet und geschlossen werden können, und die am Ende einer länglichen Halterung (50) angelenkt sind.
